# EUROPEAN PATENT SPECIFICATION

(11) **EP 3 432 778 B1**
(45) Date of publication and mention of the grant of the patent: **18.10.2023**
(21) Application number: 17769214.2
(22) Date of filing: 21.03.2017
(51) Int. Cl.: A61B 5/0531, A61B 5/01, A61B 5/00, A61B 5/053, A61H 39/02

(54) **AN APPARATUS AND METHOD TO LOCATE, MEASURE AND MONITOR INFLAMMATION OF THE SKIN'S SOFT TISSUE**
VORRICHTUNG UND VERFAHREN ZUR LOKALISIERUNG, MESSUNG, UND ÜBERWACHUNG VON ENTZÜNDUNGEN VON WEICHGEWEBE DER HAUT
APPAREIL ET PROCÉDÉ POUR LOCALISER, MESURER ET SURVEILLER UNE INFLAMMATION DES COUCHES DE TISSU MOU DE LA PEAU

(30) Priority: 21.03.2016 US 201662390094 P
(43) Date of publication of application: 30.01.2019
(73) Proprietor: Assessx Technology Ltd., Grand Forks, British Columbia V0H 1H5 (CA)
(72) Inventor: COWIE, Jocelyn Walker, Grand Forks, British Columbia V0H 1H5 (CA)
(74) Representative: McQueen, Andrew Peter
(86) International application number: PCT/CA2017/050360
(87) International publication number: WO 2017/161451

(56) References cited:
- EP-A1- 1 363 534
- EP-A2- 1 629 768
- WO-A1-90/14042
- WO-A1-91/09566
- US-A- 5 588 440
- US-A- 5 588 440
- US-A- 5 897 505
- US-A1- 2009 043 293
- US-A1- 2011 082 383
- US-A1- 2014 200 487
- US-A1- 2015 005 691
- CHRISTOPHER R REID ET AL: "Feasibility Assessment of an EVA Glove Sensing Platform to Evaluate Potential Hand Injury Risk Factors", NASA CENTER FOR AEROSPACE INFORMATION (CASI). CONFERENCE PROCEEDINGS, 12 July 2015 (2015-07-12), XP055623487, Hampton
- ROBERT TARTZ ET AL: "Effects of grip force on skin conductance measured from a handheld device : Effects of grip force on skin conductance", PSYCHOPHYSIOLOGY., vol. 52, no. 1, 23 September 2014 (2014-09-23), pages 8-19, XP055623531, US ISSN: 0048-5772, DOI: 10.1111/psyp.12237

## Description

### FIELD OF THE INVENTION

The present disclosure relates to a method and apparatus for locating and assessing soft tissue inflammations in the tissue of human beings and animals.

### BACKGROUND

It is known that the electrical resistance of skin is controlled largely through the nervous system. Canadian Patent No. 1,254,269 issued May 16, 1989 to Woodley et al. discloses a diagnostic device based upon resistance measurements of the skin which is used to detect abnormal areas of the body where there is pain or sympathetic dysfunction. U.S. Patent No. 4,966,158 issued to Honma et al. discloses a device having two probes which measures the moisture content retained in the skin both in the keratinous layer and also in the deeper layer so as to provide information as to the condition of the skin. Patent Cooperation Treaty Application No. PCT/GB90/01991 discloses a device having a common probe and a reference probe. The measurement of resistance is switched between a common probe applied to an area of skin under test and a reference probe located on the identical area on the other side of the body. A difference in the readings indicates a damaged area of the skin. These known devices measure only one parameter of the skin. Document US5588440A discloses a diagnosis apparatus which consists of a probe for contacting a desired area of skin. The probe measures the moisture content of the skin (resistance) at the area and has a device for measuring the sound generated by the skin tissue in the area. A skin lesion is indicated by a moisture content and a sound reading which are both abnormally high. The probe can also have a device that measures the temperature of the skin, indicating the presence of a lesion by detection of elevated temperature. The tip of the probe is rounded and sufficiently small so that it will cause pitting due to oedema when pressed against a damaged area of skin. The apparatus also comprises means to measure the applied force.

Cellular damage can occur due to prolonged stress, which increases build-up of metabolites and tissue ischemia. The latter build-up directly excites pain receptors and causes cellular degeneration or necrosis. Lack of use, poor posture, over use, a blow or hyperextension will cause inflammation. Inflammatory by products include histamine, bradykinin, acids, etc. are released into the capillary bed. The five cardinal signs and symptoms of inflammation-rubor, tumor, calor, and dolor, functio laesa (redness, swelling, heat, pain and loss of function) date back to Celus.

Pain causes a reflex response of muscle hoarding and/or spasms. Such a response leads to immobility and eventual wasting away or atrophy due to loss of the alternating relaxing and contracting of muscles. The muscles provide a circulatory pumping action during normal relaxation and contracting which will be ineffective on areas affected by atrophy. Ordinarily, painful areas in the skin are associated with abnormalities such as changes in temperature, and moisture, tenderness, swelling or edema, inflammation, stringiness due to fibrous tissue changes, nodules or small knotted areas, fatigue or lack of tone in the tissues, and metabolite retention characterized by crystal-like formations in the tissue. Such areas of abnormality are conventionally located by palpation.

Manual compressions, such as applied by acupressure or massage, remove the stimuli causing pain and stop the stimulation of the sweat glands and arterial vessel constriction, the primary cause of pain and degenerative tissue disorders. Such compression and massage are accompanied by the sound of the breaking up processes of metabolite and tissue by-products. Thus, factors such as moisture, sound, temperature, electrical conductivity, edema are all a function of the condition of the skin and can be used to measure the presence of areas of pain of inflammation.

In order to be able to cross correlate different types of measurements of a given area and thus obtain confirmation of the condition and a more accurate diagnosis, it would be useful to be able to measure several different parameters simultaneously.

Ultimately without controls for discrepancies data would be corrupted, for example more or less force applied when achieving a reading would change the reading of each and or any given sensor in our application.

Technically, one could first apply a device to measure resistance to a particular area and then one designed to measure moisture. However, such an approach would be impractical because not only could the condition of the area under test change from one measurement to the other, but positioning the probe on precisely the same area for both measurements would be difficult if not impractical. Secondly, many such measuring devices require measurements to be made using two separate probes applied to two separate but corresponding sides of the body.

A number of problems must be overcome in order to achieve a probe which is capable of providing measurements of a workable accuracy. For example, if one were to use infrared sensors to measure temperature, an area of the size of a quarter would be the minimum size achievable with current sensors. Thermistors would also have a limit to the area of detectability that is greater than the focal point of conductivity, which is approximately 1 millimeter (mm).

The time required to measure body temperature depends on the mass of the probe. Consequently, it is important to limit the mass of the probe in order to minimize this time.

Improved sensing of sound is also important and the shape of the sensor and how it is used is vital in detecting tissue sounds associated with crepitus and taut tendinous bands.
1) Historically massage is an empirical, tried, tested, and true practice. The applicant spent 30 years of her career relieving pain and suffering. She researched and authored numerous scientific papers. In her practice, she uses the art of massage and gets extraordinary results in the treatment of pain, disease, and trigger and acupressure point.
2) Traditionally medical practitioners are trained in palpation; they use their fingers and thumbs to detect soft tissue damage. Massage therapists palpate and massage simultaneously.
3) Devices like ultrasound, x-ray, MRIs, and CAT scans are not able to measure or localize pain causing soft tissue inflammations at its origin.

1) "Pain is the most common reason Americans turn to complementary and integrative health practices," said Josephine P. Briggs, M.D., Director of NCCAM.
2) The International Association for the Study of Pain (IASP) defines pain as an unpleasant sensory and emotional experience associated with actual or potential tissue damage, described in terms of such damage.
3) IASP supports the study of pain and translates that knowledge into improved pain relief worldwide; according to the IASP, biologists recognize that those stimuli, which cause pain, are liable to damage tissue. Since pain perception is influenced by psychosocial factors, pain that is experienced is also associated with actual or potential tissue damage. Pain is generally assessed by subjective reports, using visual analogy scales (Price et al. 1983), questionnaires, which can be converted to numeric scores (McGill, 1975) or discrete numeric scales (Price et al. 1994).
4) Early disease detection relied on the use of one's hands to evaluate the soft tissues; skilled hands can detect indications of inflammation/pain causalgia.
5) Current measurement devices do not provide sufficient evidence for measuring soft tissue inflammations that cause pain. Subjective reports of pain perception alone are considered unreliable.
6) Because there are no tools to measure pain independently, we have relied on a patient's verbal confirmation of pain, which has proven to be under evaluated. There are many obstacles to diagnose soft tissue inflammatory parameters associated with pain accurately.
7) The unifying law of pain states that the biochemical origin of all pain is inflammation and the inflammatory responses. When there is significant damage to tissue, several chemicals are released resulting in an inflammatory soup, an acidic mixture that stimulates and sensitizes the nociceptors. This is called hyperalgesia, which is Greek for super pain. Irrespective of the type of pain whether it is acute or chronic pain, peripheral or central pain, nociceptive or neuropathic pain, the underlying origin is inflammation and the inflammatory response.
8) Active signs of inflammation include the following:
   - Calor-heat is created from inflammation or lack of heat as in fibrosis
   - Dolor-pain is created when pressure is applied.
   - Functio laesa-loss of function or muscle withdrawal reflexes
   - Maturation and remodeling phase fibrosis
   - Rubor-redness is due to histamine and inflammatory chemicals
   - Sweat is how the body dissipates heat from inflammation and creates sympathetic skin response causing sweat used by the body to dissipate heat GSR measurements are used in Biofeedback and lie detectors test sympathetic skin response
   - Tissue sounds crepitus and taut muscle bands
   - Tumor-swelling oedema/tropho-edema creates a visual denting.
9) It is well known that pain is felt when pressure is applied manually or by tissue algometry. For a fibromyalgia diagnosis, a force of 4 kg or 10 lbs for a tender point to be considered "positive" the subject must state that palpation was painful. Tender is not considered painful. Studies suggest that a trigger point for myofascial pain has a reliable and reproducible pain-pressure threshold. This measure of pain still requires a subjective input patient response to indicate whether pressure stimulus is painful and is still not an objective means of determining severity of soft tissue injury.
10) Another approach to locating sites of injury is skin thermography. Skin temperature, assessed by thermography, has been found to be a sensitive test for myofascial pain syndrome. At myofascial trigger points, temperature is higher than that of surrounding locations on the skin. It has also been suggested that electrical conductivity of the skin can be used as a diagnostic measure to locate tender areas in soft tissue. Acupuncture points, known to be tender areas, appear to have higher conductivity than that of surrounding tissue. Thus, it might be possible to combine these two measures to detect the presence or absence of soft tissue injury in a more objective fashion than pain threshold or pain scores.
11) Objective measurements are difficult to obtain because skin temperature and electrical resistance of healthy tissue vary moment by moment and are not the same between individuals and both environmental and psychological factors play a role. There is no known stable norm parameter from which to obtain a base comparative measure in using existing equipment.
12) The Galvanic Skin Response (GSR) equipment measures electrical conductance in the skin, associated with the activity of the sweat glands. A very slight electrical current runs through the skin and the GSR machine measures changes in moisture produced from sweat gland ducts. The more emotionally aroused the persons the more active sweat glands and greater electrical conductivity of skin.
13) A German Professor named Tarchanoff first discovered skin conductivity around 1888. In the early 1900s, Dr. Carl Jung established that GSR measurements could track physiological arousal or stress in the body. In the 1930's Dr. Hans Selye began sharing information that could tell us about the body. These discoveries have led to the creation of many common devices, such as the polygraph. Later in the 20th century, Dr. Reinholt Voll and others identified further uses for GSR, including the monitoring of acupuncture points to determine the condition of the body's energy meridians.
14) Biofeedback and lie detection tests use GSR as emotions affect the skin's conductivity.
15) Psycho-galvanometer, biofeedback, Electro Dermal Testing (EDT), Electro Dermal Analysis (EDA), and/or GSR devices have a known potential for artifacts and spikes in the sweat and or temperature. Stable room temperature should be obtained with the bias being slightly on the warmer side 22-24-degrees Celsius. Physiological body actions like coughing, deep respiratory movements (deep sighs), sneezes, and excessive talking can all generate sweat production and thus a rest period and patient calming should happen before testing.
16) Sweat is how the body dissipates heat from inflammation creates sweat caused by sympathetic skin responses. Electro dermal activity is the property of the human body that causes continuous variation in the electrical characteristics of the skin. Since the body is in a continual state of adapting to stress and external elements there is no normal or base on which to compare either people or points of soft tissue.
17) Historically, EDA has also been known as skin conductance, GSR, Electro Dermal Response (EDR), Psychogalvanic Reflex (PGR), Skin Conductance Response (SCR), and Skin Conductance Level (SCL). The long history of research into the active and passive electrical properties of the skin by a variety of disciplines has resulted in an excess of names, now standardized to electro dermal activity.
18) The traditional theory of EDA holds that skin resistance varies with the state of sweat glands in the skin. Sweating is controlled by the sympathetic nervous system, sympathetic skin response and skin conductance is an indication of psychological or physiological arousal.
19) Electrical resistance of skin was studied with the aid of a specially designed meter that compared the resistance per surface area of small skin points with that of the surrounding skin. In a systematic study of the hands, face and ears in five subjects' low-resistance skin points were repeatedly found in characteristic loci, comparable in different individuals and symmetric about the body midline. The low-resistance skin points had diameters of 1.5 +/-0.5 mm and their borders were abrupt. On dry skin, resistance values were around 10 kilo-ohms at the center of the points but around 3 mega-ohms in the surrounding skin. Voltages could also be recorded at these points, but they proved to be result of electrode polarization reflected at these points because of their low electrical resistance. The distribution of the low points in the hands, face, and ears resembled that of classical acupuncture points.
20) If the sympathetic branch of the autonomic nervous system is highly aroused, then sweat gland activity also increases, which in turn increases skin conductance. In this way, skin conductance can be a measure of emotional and sympathetic responses.
21) More recent research and additional phenomena(resistance, potential, impedance, and admittance, sometimes responsive and sometimes apparently spontaneous) suggest this is not a complete answer, and research continues into the source and significance of EDA. The study of EDA has led to such important and vital tools the electrocardiograph (ECG) and the electroencephalograph (EEG).
22) The sympathetic branch of the autonomic nervous system is easily aroused, increasing sweat gland activity, which increases skin conductance.
23) Physiological and psychological influences on the sympathetic nervous system affect blood flow.
24) The traditional theory of Galvanic Skin Responses holds that skin resistance varies with the state of sweat glands in the skin. Sweating is controlled by the sympathetic nervous system, and skin conductance is an indication of psychological or physiological arousal.
25) More recent research and additional phenomena are sometimes apparently spontaneous. To account for changes that occur during the taking of measurements the soft tissue (including the examination process itself), we purpose a multimodal biosensors uses the concept of differential comparatives to normative tissues so that these measurement changes can be interpreted accurately.

Accordingly, it is an object of the invention to provide an improved method and apparatus for simultaneous and accurate measuring of at least two different points of the skin and at least GSR, crepitus, temperature and applied force parameters in those two points and display a comparison for the parameters at the two points.

### Summary of the Invention

According to the invention, which is exclusively defined by the appended claims, there is provided apparatus for diagnosing the skin condition of a human being or animal, which includes two probes for contacting the desired area of skin. The probe has means for measuring the conductivity of the skin at the area and means for measuring the sound produced by probes running over the skin at that area, the force applied by the probes to the skin and the temperature of the skin. The moisture content is related to the electrical resistance of the skin.

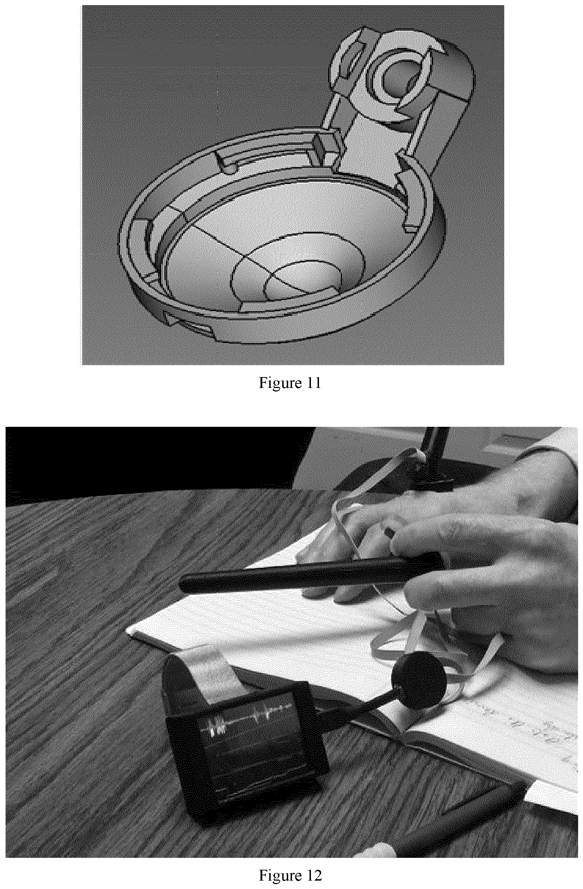

Means for measuring the conductivity or resistance of the skin may be an electrical resistance measuring device with an electrode configuration interconnecting a plurality of thermistors. For measuring the sound, a pick-up microphone is located in the probe tip (Figure 11) skin as the probe passes over a protrusion inflammation area.

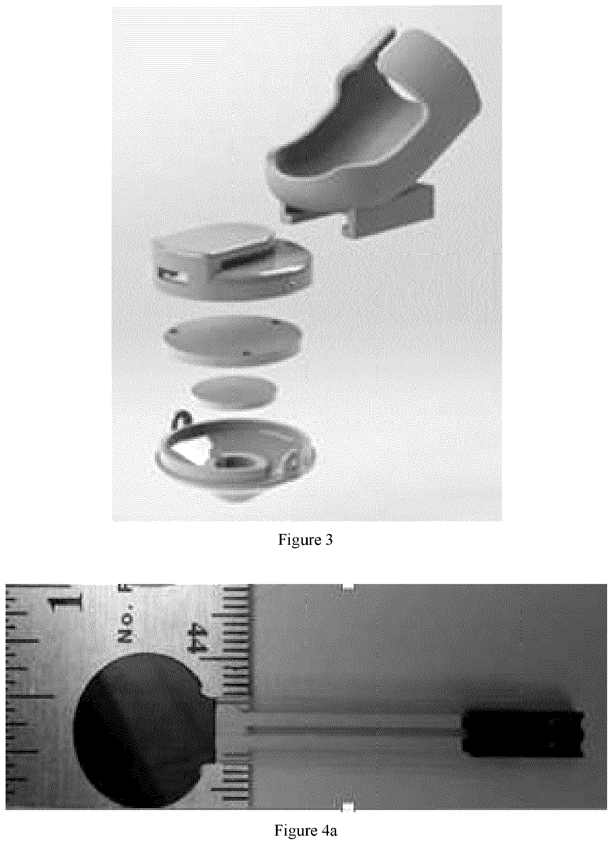

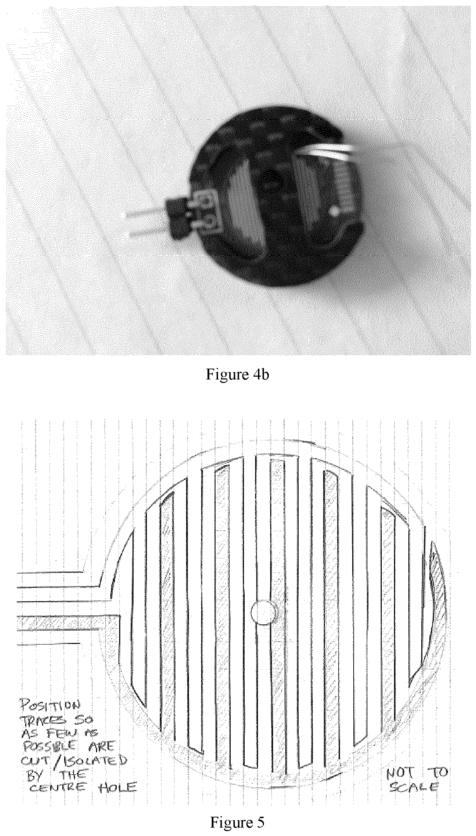

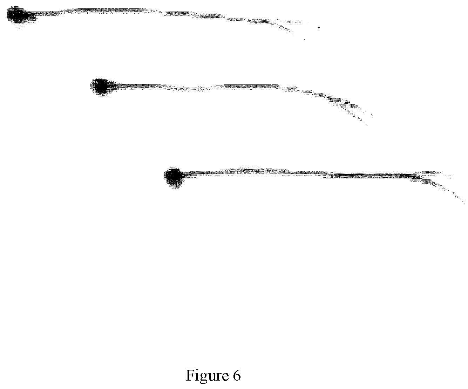

The temperature sensor for measuring the temperature of the skin may be a plurality of thermistors spaced apart over a number of probes, thermocouple located at a tip of the probe. (Figure 6)
A pressure sensor may include a strain beam coupled to the probe operative to measure applied force applied to the probe as it passes over the skin. (Figure 4)

| | |
|---|---|
| Figure 1 | ▪ Overall ABATE Qr System |
| | ▪ Colored line represent data in the display |
| | ▪ Galvanic Skin Response GSR-blue |
| | ▪ Sound-Yellow |
| | ▪ Force-Green |
| | ▪ Heat-Red-heat |
| | ▪ Purple-Patient Response Module PRM |
| | ▪ Blue Tooth/wireless communicates to software |
| Figure 2 | ▪ Miniaturized design meant to mimic fingers and/or thumbs in clinical palpation. Probes are portable and can be placed in any receptacle. |
| | ▪ Probe tips are portable be integrated into a number of various receptacle and as wearable monitor and small enough to be worn under compression garments. |
| | ▪ Pen receptacle probe with sensor tip |
| | ▪ Option for multiple bio-sensor probes design pen probe design and or into other ergonomic design, Probe# 1 and Probes #2 options for Probes #3,#4,#5 etc.... Finger and or Pen Probe sample design sensor tip is movable |
| Figure 3 | ▪ Probe assembly illustrates ceiling floor for pressure transducer |
| | ▪ Force sensor sits between floor ceiling and tip of probe pushes the sensor floor up to ceiling, pressure transducer is activated. |
| | ▪ |
| Figure 4 | ▪ Pressure transducer for detection of force applcaitions |
| Figure 5 | ▪ Interdigitated grid pattern for GSR covers a larger area of skin making GSR easier to locate |
| | ▪ Position traces so few as possible are cut/isolated by the centre hold for thermistor |
| Figure 6 | ▪ Thermistor protrudes slightly from head, which allows for indenting of the skin creating pitting edema |
| Figure 7 | ▪ Flower pot shape to house the heat sensor decreasing thermal mass. Note infrared sensor sits in from of the sensor probe set. |
| | ▪ A- thermistor slightly protruds for pitting edematous tissues . |
| Figure 8 | ▪ Size and of the heat sensor tip creates visual pitting. Fig 8- |
| Figure 9 | ▪ Conically shaped sensor head for eliciting a pain response, mobilizing crepitus, and showing visual dents from edematous tissue |
| Figure 10 | ▪ Foam padding enables the GSR sensor to lay flat on the skin surface while foam pads mimic the padding of |
| Figure 11 | Conical shaped housing for the Microphone to act as an eco-chamber for sounds from soft tissue |
| Figure 12 | ▪ LCD display |
| Figure 13 | ▪ Patient Response Module (PRM) numerical scale 1-10 |
| | ▪ |
| Figure 14 | ▪ Facial expression muscle withdraw reflex |
| | ▪ Patient Response Facial Haptic sensors move when facial expressions scaled 1-10 |
| Figure 15 | Infrared sensor sits ahead of the probe as to shoot the sensor beam in front of the sensor tip head to lead the way to the hot spots |
| Figure 16 | ▪ Patient plates with two reference points |
| | ▪ Base compared to data collected from two reference points |
| Fig 17 | ▪ Data is displayed simultaneously as a differential comparison between a minimum of two points and then is stored as a pressure point in a referential data base. |
| Figure | ▪ Schematic of basic functions |
| Figure 20 | ▪ Air cell is inflated either manually or automatically |
| | ▪ Patient Response Module can control the force being applied, the air cell will automatically release air every 30 to 90 seconds to avoid ischemia (lack of oxygen to cells) |

1) This device includes an apparatus and system to locate and measure soft tissue inflammations.
2) This device provides an objective means of determining the severity of soft tissue injury.
3) This device in an example that is not part of the claimed invention, involves a wearable monitor and a treatment-outcome software system.

Further features include:
1) In an attempt to minimize the influence of bias or prejudice on the part of the examiner, it would be effectual to provide several multi-modal parameters simultaneously between test sites to control for autonomic fluctuations. The use of two or more probes (more than two probes not being part of the claimed invention) to test differences sites stabilizes for fluctuations of the soft tissues and accounts for the ever changing the base measurement by which to compare.
2) The origin of all pain is inflammation and the inflammatory response.
3) Nociceptive neurons translate certain stimuli into action potentials that are then transmitted to more central parts of the nervous system, such as the brain. Biochemical mediators of inflammation include cytokines, neuropeptides, growth factors, and neurotransmitters. Inflammatory chemical soup consists of prostaglandins, potassium, serotonin, bradykinin, and histamine.
4) By-products of inflammatory response create measurable factors such as: temperature changes, metabolic waste and scar tissue build up, sympathetic nervous functions all a function of the condition of the soft tissue be used to measure the presence of inflammation.
5) Inflammation is caused by an opening of thousands of tiny local blood vessels in response to the interaction between cellular and chemical components and the irritation of free nerve endings.
6) The inflammatory fluid contains a high concentration of protein. This fibrinogen-containing protein is a necessary part of the body's defence mechanism against infection.
7) Excessive formation of fibrin from fibrinogen will lead to excessive scar formation. Felt as thickening, palpation elicits tissue sounds mobilizing tissue waste, the sensor probe can hear amplify and record digitized data.
8) In addition, the presence of protein increases the osmotic pressure of the tissue fluid in the damaged area, thus drawing more fluid out of the local capillaries into the tissues, causing local oedema. Inflammatory swelling starts to develop approximately two hours after the injury and may last for days and or weeks. The immediate management to control the acute inflammatory response is important to minimize the undesirable effect of the natural healing process.
9) Circulatory by-products accumulate in capillaries creating crepitus and fluid retention. Crepitus is a medical term to describe the grating, crackling, or popping sounds and sensations experienced under the skin and joints or a crackling sensation due to the presence of air in the subcutaneous tissue. Tissue sounds can also be attributed to taut muscle bands and fibrous density changes.
10) A soft tissue injury is an acute connective tissue injury that may involve muscle, ligament, tendon, capsular, and cartilaginous structures. In a sprain, strain, bruise or crush, the local network of blood vessels is damaged, and the oxygenated blood can no longer reach the tissues, resulting in cellular damage.
11) A soft tissue injury can involve muscle withdrawal reflexes a built in mechanism that allows the body's own muscles to pull away when pain is experienced by the nervous system, often with no awareness or control consciously.
12) Pain requires a conscious subject that is able to experience pain. The molecular, cellular, and systemic mechanisms that deal with the processing of pain-related information its amplification, or depression are called nociceptive, pro-nociceptive, and anti-nociceptive, respectively.
13) Pain is just one of many possible end-points of nociception. Others include but are not limited to withdrawal reflexes, vegetative and hormonal responses, and vocalization, all of which normally accompany pain experience but may under experimental and some pathological conditions be observed in the absence of pain experience, e.g., in the intact but deeply anesthetized subject or in inflammationed animals.
14) A patient can verbally express pain on a numerical scale however; studies have shown this is unreliable. Intensive care patients are people suffering from serious injuries or diseases. These people receive highly specialized care and medical attention, and are under continuous observation and monitoring.
15) It is important to note that ICU patients may not be able to respond to a voice or tactile stimulation. Many patients in the ICU are in breathing tubes, which prevents them from communicating the pain that they are experiencing as well.
16) Health care providers need to provide cost effective pain therapies that will enable the therapist to gain the information needed to deploy treatments and objectively monitor results. It quickly locates inflammation and any soft tissue damage. R.I.C.E. is the acronym for Rest, Ice, Compression, and Elevation; commonly prescribed for patient with acute soft tissue injury.
17) The purposed apparatus uses the complete thesis understanding bio physiological functions of the soft tissue to obtain a multiple sensors electronic measures to locate differential comparative areas that indicate inflammations according to the scope defined exclusively by the appended claims.
18) In an example, that is not part of the claimed invention, a centralized database would enable analysis of outcomes from therapies. Data can also be exported in anonymous nationwide comparison outcomes analytics of pain therapies.
19) In order to overcome the physiologically adaptations to psychological and environment stress and factors including the applying pressure to pain points cause continual physiological adaptations and adjustment in the systemic and local measurements, so there is no such thing as normative data to compare to in the collection of the skins environmental adaptive attributes.
20) Physiological and psychological influences on the sympathetic nervous system controls the blood flow to the inflamed tissues as does systemic sympathetic nervous function creating he sympathetic branch of the autonomic nervous system is easily aroused increasing sweat gland activity increases, this in turn increases skin conductance and blood flow.
21) The traditional theory of Galvanic Skin Responses holds that skin resistance varies with the state of sweat glands in the skin. Sweating is controlled by the sympathetic nervous system, and skin conductance is an indication of psychological or physiological arousal.
22) More recent research and additional phenomena are sometimes apparently spontaneous. To account for changes that occur during the taking of measurements the soft tissue, including the examination process itself, we purpose multimodal biosensors that use the concept of differential comparatives to normative tissues so that these measurement changes can be interpreted accurately.
23) In order to overcome the physiologically adaptations to psychological and environment stress and factors including applying pressure to pain points which causes continual adaptation and adjusting, so there is no such thing as normative data to compare to in the collection of the skins environmental elements.

### Brief Description of the Drawings

Further features and advantages will be apparent from the following detailed description, given by way of example, of a preferred embodiment taken in conjunction with the accompanying drawings, wherein:
Figure 1 is an elevation of a massage therapist wrist with an LCD display and the fingers of a hand holding the instrument, which carries a probe set for differential measurements of temperature, sound, moisture (GSR) and applied force
Figures 2a and 2b is a first perspective view of the probe miniaturized design that can be portable into other receptacles.
Figure 3 is an exploded view of the sensor unit
Figures 4a and 4b is a first perspective view of the pressure transducer sensor unit
Figure 5 is a perspective view of the GSR sensor and sensor unit with the concentric, interdigitated electrode array and temperature sensors
Figure 6 is a perspective view of a thermistor that is mounted into the probe tips, it lies in a slight recess within the flower pot shape (see Figure 7) ;
Figures 7a and 7b a function of probe for a non-thermal flower pot shape to eliminate the conductive temperature measurements being absorbed by a mass, this shows the protrusion shaped sensor tip that houses thermistors in the very central position, this also permits and or allows for pitting the tissue ( see Figure 8) ;
Figures 8a and 8b as it passes through a inflammation both before and after massage; as a function of the probe, when pressure and or friction massage is applied on the skin, the protruded shape tip elicits or helps create sounds from inflamed soft tissues, pitting is also a visible sign (camera can take an image and store it in patient file record) of inflamed soft tissue created by the protruded shape
Figure 9 exact dimensions for conical shaped head of sensor for eliciting pain with pressure and crepitus sounds with massaging action
Figure 10 is a foam padding that cushions the sensor tip to feel more like a finger, it also stops external sounds from entering the conical shaped housing for the microphone as a function of probe position as it passes over an inflammation both before and after massage
Figure 11 is a conical shaped housing hosts a flower pot base where the microphone sits and is isolated from external sounds by a ceiling (see Figure 3) and by foam pad (see Figure 10).
Figure 12 is a LCD display

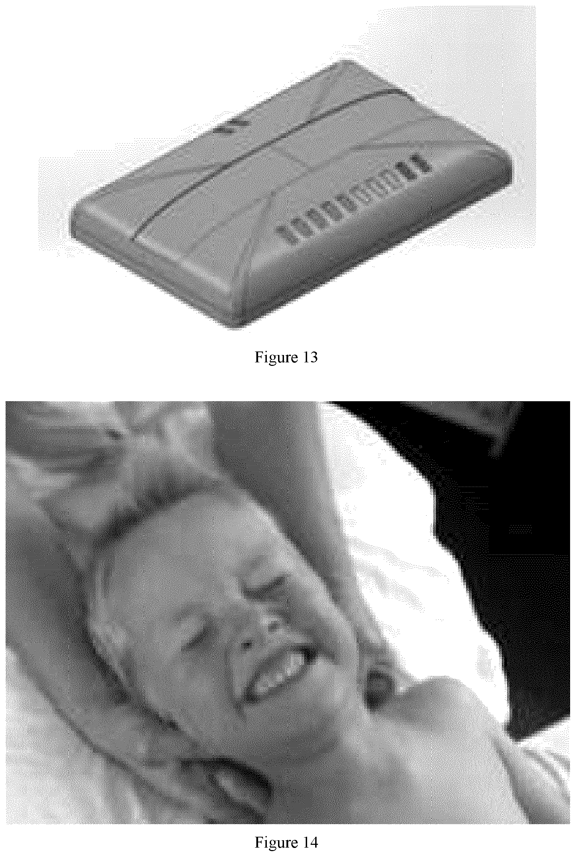

Figure 13 is a Patient Response Module a handheld device in the patient's hand used blind to the sensor data that allows the patient to rate their pain as pressure is being applied without need for verbal communication
Figure 14 is a facial expression showing how muscles react to pain whereby haptic sensors can be placed on said muscles to supplement the patient response module (see Figure 13) when a patient is unable to verbalize pain
Figure 15 is an infrared sensor that sits in front of the probe tip to lead therapist toward the inflammation.
Figure 16 is software that allows mapping of pain points on digitized images and stores data related those points.
Figure 17 is software that allows data to be displayed as a differential from two or more probes and simultaneously displays input from the patient response module.
Figure 18 is a schematic of the basic function of the probes and Bluetooth wireless communication

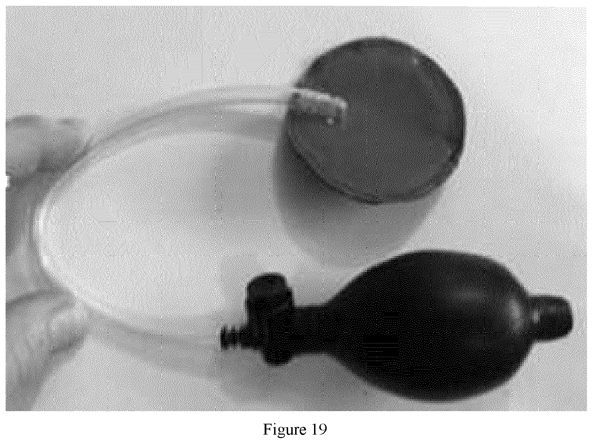

Figure 19 is an air cell that sits on top of the miniaturized sensor tip and can be inflated manually or automatically and is controlled by the patient including using the patient response module wirelessly (see Figure 15).
Figure 20 is a sectional view of a sensor with an inflatable air cell.

### Detailed Description With Reference to the Drawings

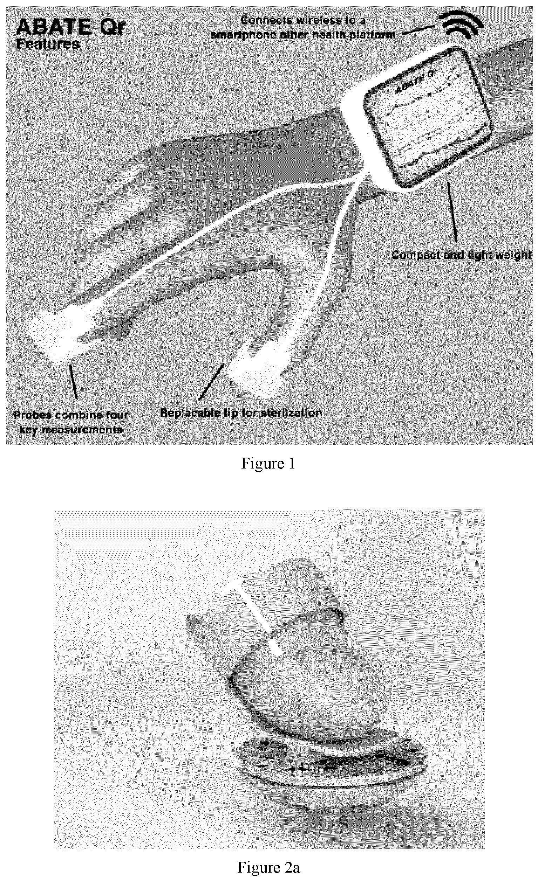

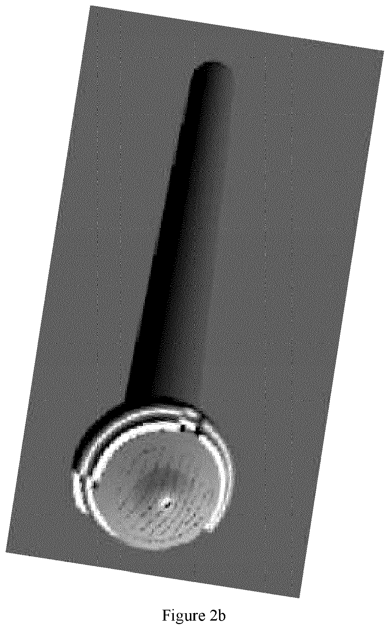

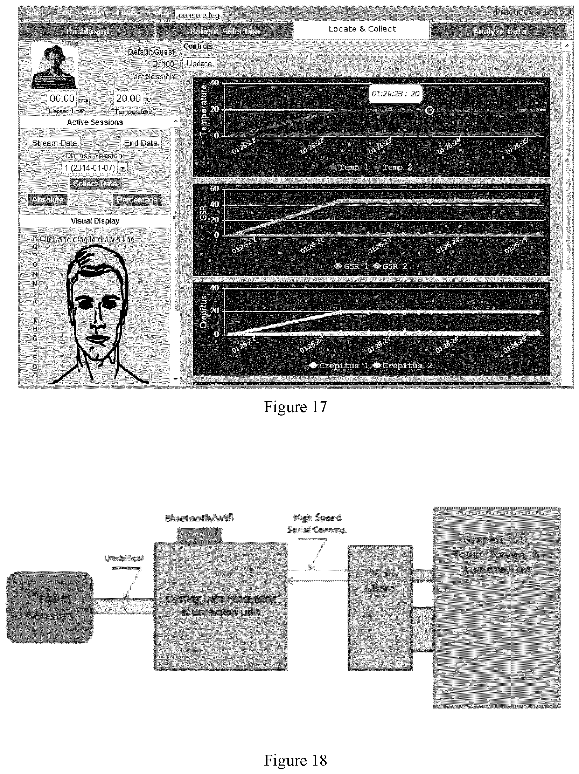

Referring to Figures 1 and 2 there is shown a sensor instrument on the finger receptacle. The probe can be installed in the bottom any receptacle Figure 2. Figure 1 shows an LCD display screen. A separate infrared sensor (Figure 17) may detect infrared Z(IR) radiation from the skin boundary forwardly of the sensor instrument. Such an arrangement has the advantage of increased IR sensitivity, a constant elevation reference, and no influence from lens warming or lens absorption of IR body energy.

The probe tips house a circuit board and other components including a strain beam (Figure 4) that has downward force applied. This will exert a bending movement on beam (Figure 4) and result in a strain that is recorded by load cells. Thus, the strain beam provides a measure of the force applied user or air cell (Figure 20).

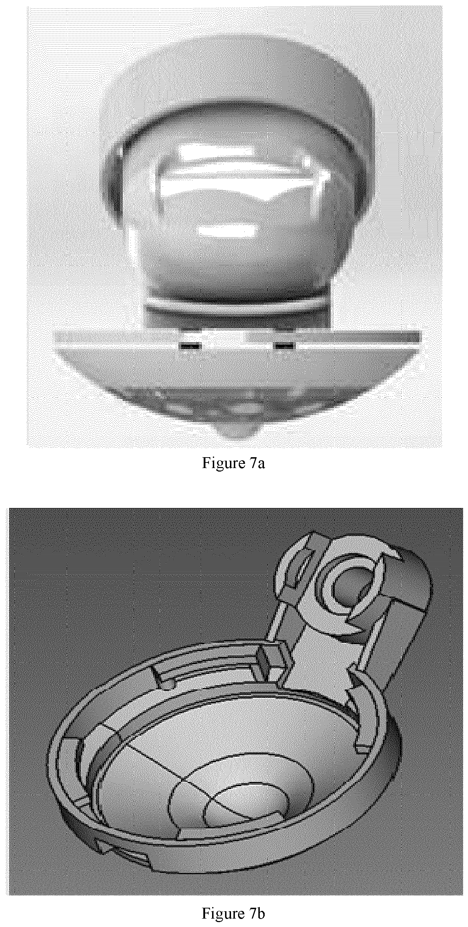

The exploded view of Figure 3 shows the various components of the probe including thermistors on its tip ( Figure 7) located in the center. When the central thermistor is over an inflammation, it records the temperature of the point of inflammation while the other thermistors record a base temperature a short distance away from the inflammation.

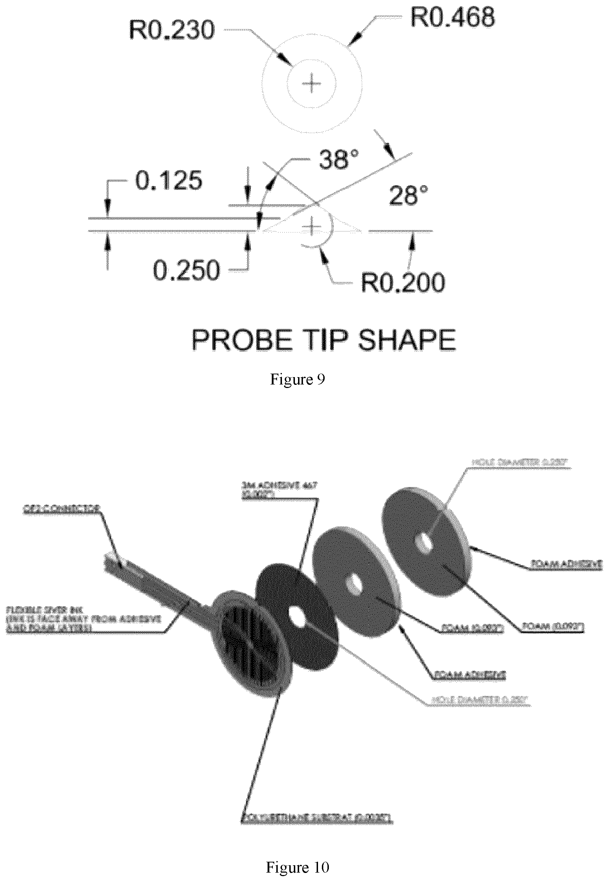

A printed GSR sensor (Figure 5) of interconnected silver conductive electrodes formed into a ring, fits over the rest of the sensor tip on top of foam pads (see figure 10) the centre is left open to accommodate the thermistor sensor tip. When a voltage is applied across the silver the differences between probes is established. Typically the focal point of conduction on the skin is approximately 1 mm. Thus, using only a thermistor to find the focal point would be inaccurate due to the much larger dimensions of the thermistor. When the probe presses against the skin, any inflammation will usually be between a set of concentric electrodes. Thus the resistance between the inflammation electrodes will be the body resistance xR1 of the skin on one side of the inflammation, the inflammation resistance R2 of the inflammation and the body resistance yR1 of the skin on the other side of the inflammation, where x + y = 1. As the inflammation gets closer to the center of the concentric electrodes, and the body resistance of the skin therefore becomes less in inflamed areas. Thus, by monitoring the resistance as the probe moves over the skin, a user can tell if the inflammation is moving towards the center. When the point of inflammation is over the center of pain the display screen shows the difference.

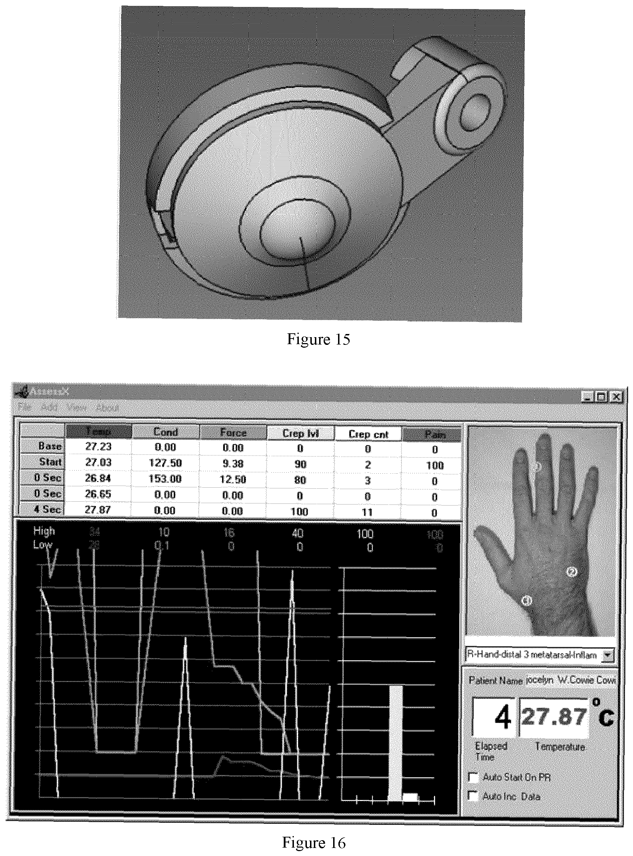

An IR sensor receives filtered IR light passing through the lens (Figure 15).

A high sensitivity microphone (Figure 11) is mounted in the center of the probe tip under the top base printed circuit board (PCB). The microphone (Figure 11) detects the sound of the display screen moving over the skin.

The temperature component of the probe ( Figure 6) is formed by thermistors, which contact the skin and sense the temperature between probe tips. The precise center of the inflammation can be determined using the measurements mentioned above.

Prior to using the instrument (Figure 1), a massage therapist or other professional locates the point of inflammation manually and then measures the conductivity of the skin a few inches away from the point of inflammation. This measurement of the body resistance provides a base measurement for moisture content of the skin. Further measurements near the inflamed area are then compared to the base measurement to give a relative measure of moisture content.

The simultaneous development of signals which correspond to moisture content (GSR), temperature, and sound allow all three of these factors to be cross correlated to confirm an indicated condition by any one of them and to more accurately define the nature and extent of the condition. The strain beam measurement allows a user to monitor and control the amount of pressure being applied. Pressure must be equally applied between probes to maintain consistency for stabilizing other measures. This is extremely important otherwise pressure will alter the viability of other readings.

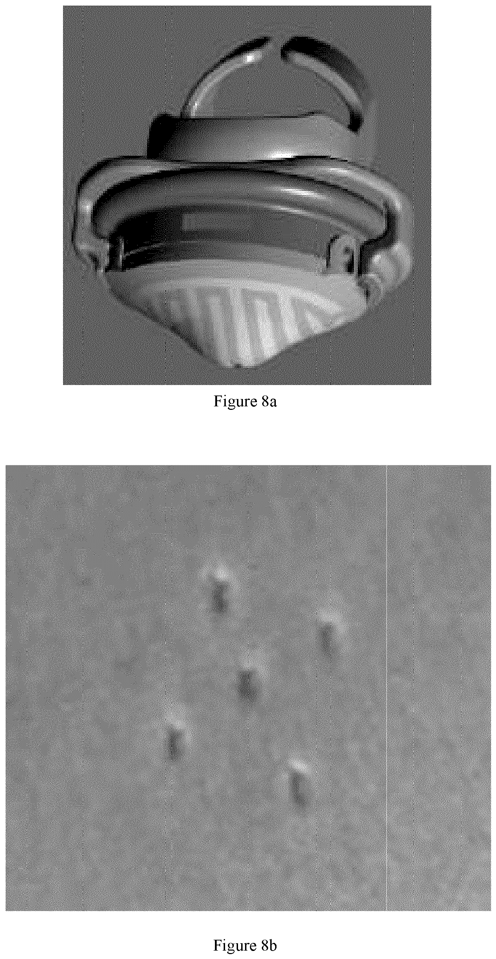

One may determine the pressure required to cause pitting edema, another sign of inflammations. As protrusion inflammations develop and become fibrous, they create a "speed bump" to the probe (Figure 8) as it passes over the area of the protrusion inflammation.

Applied pressure rises as the pressure sensor on the probe presses over inflammation pitting of soft tissue can be a result ( Figure 8). Digital images can be imported into patient record.

Figures 16 and 17 show graphs of the readings of temperature, resistance, and sound as one progresses towards, over and then away from an inflammation. The same figures show readings of inflammation after applying massage. Thus, the probe allows the massage therapist to both apply massage and determine the effect of the massage on an inflammation to a quantifiable extent. A therapist can use the sound output to rapidly locate suspected damaged areas of the skin and then to confirm the damage using the other readings of temperature, moisture content and resistance. Any extraneous readings in any one or more of the factors of temperature, moisture, and sound can be checked as to their origin by comparing them to the readings for the other factors.

Figure 20 shows an inflatable air cell that can be placed under a tensile bandage or other means of securing it on top of the sensor probe. The probe is used to locate the precise point of inflammation and then the air cell is secured in place to apply pressure to the probe and air cell by pumping compressed air through the valve. Expansion of the air cell against the tensile force of a bandage causes the tip of the probe to press against the point of inflammation. This function can be done automatically or manually and the patient can control the amount of force being applied by the air cell either manually or wirelessly via the patient response module.

Accordingly, while this invention has been described with reference to illustrative embodiments, this description is not intended to be construed in a limiting sense.

## Claims

1. Apparatus for monitoring inflammation of the skin of a patient during palpation of the patient by a therapist, the apparatus comprising:
first and second probes configured to contact a first and a second area of the patient's skin, each of the probes comprising a printed galvanic skin response (GSR) sensor configured to measure conductivity or resistance of the skin, a microphone configured to measure crepitus produced by movement of the probe over the skin, a pressure sensor operative to measure a force by which the probe is being pushed against the skin, and a temperature sensor configured to measure the temperature of the skin, wherein:
the GSR sensors comprise electrodes arranged in concentric , interdigitated electrode array;
the sensors of the first and second probes each connected to a data processing and collection unit comprising means configured to monitor outputs of the sensors of the first and second probes and a display configured to simultaneously display the outputs of the corresponding sensors of the first and second probes for comparison.

2. The apparatus according to claim 1 wherein the data processing and collection unit is configured to measure a first electrical conductivity or resistance sensed by the GSR sensor of the first probe and a second electrical conductivity or resistance sensed by the GSR sensor of the second probe and comprises means configured to display a difference between the first electrical conductivity or resistance and the second electrical conductivity or resistance.

3. The apparatus according to claim 2 wherein the temperature sensor of each of the first and second probes is centrally located relative to the GSR sensor of the probe and the data processing and collection unit is configured to determine a first temperature sensed by the temperature sensor of the first probe and a second temperature sensed by the temperature sensor of the second probe and to display on the display a comparison of the first and second temperatures.

4. The apparatus according to any one of claims 1 to 3 wherein the data processing and collection unit is configured to determine a first crepitus level sensed by the microphone of the first probe and a second crepitus level sensed by the microphone of the second probe and to display on the display a comparison of the first and second crepitus levels.

5. The apparatus according to any one of claims 1 to 4 wherein the force sensor comprises a strain beam and load cells coupled to measure forces applied to the strain beam.

6. The apparatus according to any one of claims 1 to 5 wherein each of the first and second probes is detachably connected to a finger receptacle comprising a socket configured to receive a finger or thumb of a user.

7. The apparatus according to any one of claims 1 to 6 wherein at least one of the first and second probes further comprises an infrared sensor.

8. The apparatus according to any one of claims 1 to 7 further comprising a patient response module comprising an input by way of which the patient can rate a sensation of pain.

9. The apparatus according to claims 1 to 8 further comprising an inflatable air cell and an automatic pressure control connected to periodically release and reapply pressure, the inflatable air cell positionable on top of one of the first and second probes.

10. A method for monitoring inflammation of a patient, the method comprising:
applying first and second probes to the skin of the patient, each of the probes comprising a printed galvanic skin response (GSR) sensor, a microphone, a pressure sensor and a temperature sensor;
measuring conductivity or resistance of the skin at the site of each probe using the respective GSR sensor;
measuring crepitus at the site of each probe using the respective microphone;
measuring a force by which each probe is pushed against the skin using the respective pressure sensor;
measuring skin temperature at the site of each probe using the respective temperature sensor;
displaying on a display a comparison of an output of the sensors of the first probe and an output of the sensors of the second probe.

11. The method according to claim 10 wherein skin temperature is measured at locations central with respect to the GSR sensors of the first and second probes respectively.

## Patentansprüche

1. Vorrichtung zur Überwachung von Entzündung der Haut eines Patienten während der Palpation des Patienten durch einen Therapeuten, wobei die Vorrichtung umfasst:
eine erste und eine zweite Sonde, die konfiguriert sind, einen ersten und einen zweiten Bereich der Haut des Patienten zu kontaktieren, wobei jede der Sonden einen gedruckten galvanischen Hautreaktionssensor (GSR-Sensor) umfasst, der konfiguriert ist, die Leitfähigkeit oder den Widerstand der Haut zu messen, ein Mikrofon, das konfiguriert ist, die durch die Bewegung der Sonde über die Haut erzeugte Krepitation zu messen, einen Drucksensor, der dazu dient, eine Kraft zu messen, mit der die Sonde gegen die Haut gedrückt wird, und einen Temperatursensor, der konfiguriert ist, die Temperatur der Haut zu messen, wobei:
die GSR-Sensoren Elektroden, die in einer konzentrischen, ineinandergreifenden Elektrodenanordnung angeordnet sind, umfassen;
wobei die Sensoren der ersten und zweiten Sonde jeweils mit einer Datenverarbeitungs- und -erfassungseinheit verbunden sind, die Mittel, die zum Überwachen der Ausgaben der Sensoren der ersten und zweiten Sonde konfiguriert sind, und eine Anzeige, die zum gleichzeitigen Anzeigen der Ausgaben der entsprechenden Sensoren der ersten und zweiten Sonde zum Vergleich konfiguriert ist, umfasst.

2. Vorrichtung gemäß Anspruch 1, wobei die Datenverarbeitungs- und - erfassungseinheit konfiguriert ist, eine erste elektrische Leitfähigkeit oder einen ersten elektrischen Widerstand, erfasst durch den GSR-Sensor der ersten Sonde, und eine zweite elektrische Leitfähigkeit oder einen zweiten elektrischen Widerstand, erfasst durch den GSR-Sensor der zweiten Sonde, zu messen, und Mittel umfasst, die konfiguriert sind, eine Differenz zwischen der ersten elektrischen Leitfähigkeit oder dem ersten elektrischen Widerstand und der zweiten elektrischen Leitfähigkeit oder dem zweiten elektrischen Widerstand anzeigen.

3. Vorrichtung gemäß Anspruch 2, wobei der Temperatursensor sowohl der ersten als auch der zweiten Sonde in Bezug auf den GSR-Sensor der Sonde zentral gelagert ist und die Datenverarbeitungs- und -erfassungseinheit konfiguriert ist, eine die von dem Temperatursensor der ersten Sonde erfasste erste Temperatur zu bestimmen und eine von dem Temperatursensor der zweiten Sonde erfasste zweite Temperatur zu bestimmen und auf der Anzeige einen Vergleich der ersten und zweiten Temperatur anzuzeigen.

4. Vorrichtung gemäß einem der Ansprüche 1 bis 3, wobei die Datenverarbeitungs- und -erfassungseinheit konfiguriert ist, einen ersten Grad von Krepitation, der vom Mikrofon der ersten Sonde erfasst wird, und einen zweiten Grad von Krepitation, der vom Mikrofon der zweiten Sonde erfasst wird, zu bestimmen und auf der Anzeige einen Vergleich des ersten und zweiten Grades von Krepitation anzuzeigen.

5. Vorrichtung gemäß einem der Ansprüche 1 bis 4, wobei der Kraftsensor einen Dehnungsbalken und Kraftmesszellen umfasst, die zum Messen der auf den Dehnungsbalken angewendeten Kräfte gekoppelt sind.

6. Vorrichtung gemäß einem der Ansprüche 1 bis 5, wobei sowohl die erste als auch die zweite Sonde lösbar mit einer Fingeraufnahme verbunden ist, die eine Buchse umfasst, die zur Aufnahme eines Fingers oder Daumens eines Benutzers konfiguriert ist.

7. Vorrichtung gemäß einem der Ansprüche 1 bis 6, wobei mindestens eine von der ersten und zweiten Sonde ferner einen Infrarotsensor umfasst.

8. Vorrichtung gemäß einem der Ansprüche 1 bis 7, ferner umfassend ein Patientenreaktionsmodul, das einen Eingang umfasst, über den der Patient ein Schmerzempfinden bewerten kann.

9. Vorrichtung gemäß den Ansprüchen 1 bis 8, ferner umfassend eine aufblasbare Luftzelle und eine automatische Drucksteuerung, die so angeschlossen ist, dass der Druck periodisch abgelassen und erneut angelegt wird, wobei die aufblasbare Luftzelle auf einer von der ersten und zweiten Sonde positionierbar ist.

10. Verfahren zur Überwachung einer Entzündung eines Patienten, wobei das Verfahren umfasst:
Anbringen einer ersten und zweiten Sonde an der Haut des Patienten, wobei jede der Sonden einen gedruckten galvanischen Hautreaktionssensor (GSR-Sensor), ein Mikrofon, einen Drucksensor und einen Temperatursensor umfasst;
Messen der Leitfähigkeit oder des Widerstands der Haut an der Stelle jeder Sonde unter Verwendung des jeweiligen GSR-Sensors;
Messen der Krepitation an der Stelle jeder Sonde unter Verwendung des jeweiligen Mikrofons;
Messen einer Kraft, mit der jede Sonde gegen die Haut gedrückt wird, unter Verwendung des jeweiligen Drucksensors;
Messen der Hauttemperatur an der Stelle jeder Sonde unter Verwendung des jeweiligen Temperatursensors;
Anzeigen eines Vergleichs einer Ausgabe der Sensoren der ersten Sonde und einer Ausgabe der Sensoren der zweiten Sonde auf einer Anzeige.

11. Verfahren nach Anspruch 10, wobei die Hauttemperatur an Stellen gemessen wird, die in Bezug auf die GSR-Sensoren der ersten bzw. zweiten Sonde zentral sind.

## Revendications

1. Appareil pour la surveillance d'une inflammation de la peau d'un patient pendant la palpation du patient par un thérapeute, l'appareil comprenant :
des première et seconde sondes conçues pour venir en contact avec une première et une seconde zone de la peau du patient, chacune des sondes comprenant un capteur de réponse galvanique cutanée (GSR) imprimé conçu pour mesurer la conductivité ou la résistance de la peau, un microphone conçu pour mesurer la crépitation produite par le déplacement de la sonde sur la peau, un capteur de pression fonctionnant pour mesurer une force avec laquelle la sonde est appuyée contre la peau et un capteur de température conçu pour mesurer la température de la peau, dans lequel :
les capteurs de GSR comprennent des électrodes agencées en réseau d'électrodes interdigitées concentriques ;
les capteurs des première et seconde sondes sont chacun connectés à une unité de collecte et de traitement de données comprenant un moyen conçu pour surveiller des sorties des capteurs des première et seconde sondes et un afficheur conçu pour afficher simultanément les sorties des capteurs correspondants des première et seconde sondes pour comparaison.

2. Appareil selon la revendication 1 dans lequel l'unité de collecte et de traitement de données est conçue pour mesurer une première conductivité ou résistance électrique détectée par le capteur de GSR de la première sonde et une seconde conductivité ou résistance électrique détectée par le capteur de GSR de la seconde sonde et comprend un moyen conçu pour afficher une différence entre la première conductivité ou résistance électrique et la seconde conductivité ou résistance électrique.

3. Appareil selon la revendication 2 dans lequel le capteur de température de chacune des première et seconde sondes a un emplacement central par rapport au capteur de GSR de la sonde et l'unité de collecte et de traitement de données est conçue pour déterminer une première température détectée par le capteur de température de la première sonde et une seconde température détectée par le capteur de température de la seconde sonde et pour afficher sur l'afficheur une comparaison des première et seconde températures.

4. Appareil selon l'une quelconque des revendications 1 à 3 dans lequel l'unité de collecte et de traitement de données est conçue pour déterminer un premier niveau de crépitation détecté par le microphone de la première sonde et un second niveau de crépitation détecté par le microphone de la seconde sonde et pour afficher sur l'afficheur une comparaison des premier et second niveaux de crépitation.

5. Appareil selon l'une quelconque des revendications 1 à 4 dans lequel le capteur de force comprend une jauge de contrainte et des cellules de charge couplées pour mesurer des forces appliquées à la jauge de contrainte.

6. Appareil selon l'une quelconque des revendications 1 à 5 dans lequel chacune des première et seconde sondes est connectée de manière détachable à un logement pour doigt comprenant une emboîture conçue pour recevoir un doigt ou le pouce d'un utilisateur.

7. Appareil selon l'une quelconque des revendications 1 à 6 dans lequel au moins l'une des première et seconde sondes comprend en outre un capteur infrarouge.

8. Appareil selon l'une quelconque des revendications 1 à 7 comprenant en outre un module de réponse du patient comprenant une entrée au moyen de laquelle le patient peut évaluer une sensation de douleur.

9. Appareil selon les revendications 1 à 8 comprenant en outre une cellule pneumatique gonflable et une commande de pression automatique connectées pour relâcher et réappliquer périodiquement de la pression, la cellule pneumatique gonflable pouvant être positionnée au-dessus de l'une des première et seconde sondes.

10. Procédé pour la surveillance d'une inflammation d'un patient, le procédé comprenant :
l'application de première et seconde sondes sur la peau du patient, chacune des sondes comprenant un capteur de réponse galvanique cutanée (GSR) imprimé, un microphone, un capteur de pression et un capteur de température ;
la mesure de la conductivité ou résistance de la peau au niveau du site de chaque sonde à l'aide du capteur de GSR respectif ;
la mesure de crépitation au niveau du site de chaque sonde à l'aide du microphone respectif ;
la mesure d'une force avec laquelle chaque sonde est appuyée contre la peau à l'aide du capteur de pression respectif ;
la mesure de température de la peau au niveau du site de chaque sonde à l'aide du capteur de température respectif ;
l'affichage sur un afficheur d'une comparaison d'une sortie des capteurs de la première sonde et d'une sortie des capteurs de la seconde sonde.

11. Procédé selon la revendication 10 dans lequel la température de la peau est mesurée au niveau d'emplacements centraux par rapport aux capteurs de GSR des première et seconde sondes respectivement.
